# EUROPEAN PATENT APPLICATION

(11) **EP 3 756 478 A1**
(43) Date of publication of application: **30.12.2020**
(21) Application number: 20190866.2
(22) Date of filing: 03.02.2017
(51) Int. Cl.: A23L 33/19, A23L 33/18, A61K 38/00, A23K 20/147, A61K 38/43, A61P 19/02, A23L 2/66

(54) **FOOD COMPOSITION FOR IMPROVING CARTILAGE FUNCTION**

(30) Priority: 04.02.2016 JP 2016020281
(62) Document number(s) of the earlier application(s) in accordance with Art. 76 EPC: 17747551.4 / 3 412 301
(71) Applicant: Megmilk Snow Brand Co. Ltd., Sapporo-shi, Hokkaido 065-0043 (JP)
(72) Inventor: KATO, Ken, Hokkaido, Tokyo 065-0043 (JP); ISHIDA, Yuko, Hokkaido, Tokyo 065-0043 (JP); MORITA, Yoshikazu, Hokkaido, Tokyo 065-0043 (JP); WADA, Masahiro, Hokkaido, Tokyo 102-0094 (JP); NAKATANI, Sachie, Hokkaido, Tokyo 102-0094 (JP); UEDA, Hiroya, Hokkaido, Tokyo 102-0094 (JP)
(74) Representative: Wakerley, Helen Rachael

(57) **Abstract**

The present invention provides a food product composition for improving cartilage functions useful for prevention and treatment of a variety of joint diseases. The present invention also provides a drink or food product for improving cartilage functions, a nutritive composition for improving cartilage functions, and a livestock food for improving cartilage functions, these products comprising the food product composition for improving cartilage functions.

The food product composition for improving cartilage functions comprises at least one effective ingredient selected from the group consisting of cystatin, HMG-like protein, and/or degraded products thereof. Since the food product composition for improving cartilage functions according to the present invention can be daily ingested with safety for a long time and has a noticeable effect of repairing or regenerating deformed or lost cartilages, the food product composition is useful for prevention and treatment of a variety of joint diseases.

## Description

### TECHNICAL FIELD

The present invention relates to a food product composition for improving cartilage functions. The composition has an action of promoting the proliferation of chondrocytes and/or retarding the calcification of the chondrocytes, has a noticeable effect of modifying or regenerating deformed or lost cartilages, and is useful in prevention and treatment of a variety of joint diseases. The present invention also relates to a drink or food product for improving cartilage functions, a nutritive composition for improving cartilage functions, and a livestock food for improving cartilage functions, these products comprising the food product composition for improving cartilage functions.

### BACKGROUND ART

Japan has an average life expectancy of more than 80 years, and has reached a super-aged society, where about one in four persons is 65 years old and over. With such ageing, the prevalence of locomotive organ disorders has been increasing. In 2007, the Japanese Orthopaedic Association Corporation has suggested a new term "locomotive syndrome" to promote a change in attitudes of people and medical practitioners toward the maintenance and increase of the heath of the locomotive organs. The locomotive syndrome represents a state of care needing caused by or its risk increased by dysfunction of the locomotive organs. The locomotive organs generally include organs which support and move bodies, such as bones, joints, ligaments, spines, spinal cord, muscles, tendons, and peripheral nerves. Examples of representative diseases and dysfunctions observed in these locomotive organs include osteoporosis, sarcopenia, and osteoarthritis.

Osteoarthritis is the most popular joint disease, and is expected to be found in 20 to 30% of people of fifty years and older. In particular, the number of patients has been increasing, and a large number of patients having osteoarthritis are forced to put up with inconveniences in daily life. The disease is believed to come from degeneration of joint forming elements caused by ageing and genetic factors, or loads to joints generated by obesity, work, or sports. A decrease in cartilages, which occurs at the epiphyseal surfaces supporting movement of bones, leads to deformation or loss of the cartilages. Such deformation or loss inhibits smooth movements of the joints, causing osteoarthritis.

Because the cartilage has no blood vessels and no nerve cells, it is believed that the cartilage, once damaged, is difficult to spontaneously recover. For this reason, physiotherapy, such as heat therapy or tugging, or palliative therapy using analgesics or anti-inflammatory drugs are used in light cases of osteoarthritis whereas injection of hyaluronic acid into joints or surgical replacement with artificial joints is performed in serious cases thereof. To improve the quality of life (QOL) of the patient through complete cure of osteoarthritis, because of the characteristics of the disease, the patient should daily and safely take a food product or effective ingredients contained in the food product for a long time to repair or regenerate the deformed or lost cartilage of the patient.

The cartilage is composed of chondrocytes differentiated from mesenchymal stem cells and a cartilage matrix of collagen, hyaluronic acid, and proteoglycan produced by the chondrocytes. Usually, the cartilage is calcificated after proliferation, ageing, and hypertrophy of chondrocytes, while the cartilage matrix is being degenerated to be transformed to bone tissues. In joints and intervertebral disks, the chondrocytes keep their characteristics throughout life; hence the cartilage provides smooth mobility to the body without transformation to a bone. Accordingly, in order to repair or regenerate the deformed or lost cartilage for complete cure osteoarthritis, it is important to promote the proliferation of the chondrocytes present in the cartilage and the production of the cartilage matrix in the chondrocytes and to prevent the transformation of the cartilage to a bone through retardation of calcification of the chondrocytes.

Food product ingredients promoting the proliferation of chondrocytes present in the cartilage and production of the cartilage matrix by the chondrocytes or retarding the calcification of the chondrocytes to prevent the transformation of the cartilage to a bone are disclosed: for example, glucosamine hydrochloride promoting the production of the cartilage matrix by the chondrocytes and retarding the calcification of the chondrocytes (Non Patent Document 1), and a dipeptide derived from collagen, prolylhydroxyproline, promoting the production of the cartilage matrix by the chondrocytes and retarding the calcification of chondrocytes (Non Patent Document 2).

Milk and dairy products are food products which have been eaten since ancient times and can be daily taken with safety for a long time. It is known that the milk basic protein fractions contained therein and their degraded products can promote the production of the cartilage matrix. It is also known that lactoferrin can promote the proliferation of chondrocytes and retard the calcification of the chondrocytes (Patent Documents 1 and 2, Non-Patent Document 3).

### RELATED ART

### Patent Documents

Patent Document 1: WO2010/058679
Patent Document 2: WO2013/164992

### Non Patent Documents

Non Patent Document 1: Nakatani et al., Biological & Pharmaceutical Bulletin, 30: 433-438 (2007)
Non Patent Document 2: Nakatani et al., Osteoarthritis and Cartilage, 17: 1620-1627 (2009)
Non Patent Document 3: Takayama et al., Biometals, 23: 477-484 (2010)

### SUMMARY OF INVENTION

### TECHNICAL PROBLEM

Although lactoferrin is a food product which can be daily taken with safety for a long time, promote the proliferation of chondrocytes, and retard the calcification of the chondrocytes, it is somewhat disadvantageous in stability and flavor. For this reason, a food product composition for improving cartilage functions having improved characteristics has been required to solve these problems.

An object of the present invention is to provide a food product composition for improving cartilage functions which can be daily taken with safety for a long time, has an action of promoting the proliferation of chondrocytes and/or an action of retarding the calcification of the chondrocytes, has a noticeable effect of modifying or regenerating a deformed or lost cartilage, and is useful for prevention and treatment in a variety of joint diseases. Another object of the present invention is to provide a drink or food product for improving cartilage functions, a nutritive composition for improving cartilage functions, and a livestock food for improving cartilage functions which contain the food product composition for improving cartilage functions.

### SOLUTION TO PROBLEM

The present invention relates to the following aspects:
Aspect (1) A food product composition for improving cartilage functions, comprising at least one effective ingredient selected from the group consisting of lactoperoxidase, cystatin, high mobility group (HMG)-like protein, and degraded products thereof.
Aspect (2) The food product composition for improving cartilage functions according to Aspect (1), wherein the lactoperoxidase, the cystatin, and the HMG-like protein are derived from mammal milk.
Aspect (3) The food product composition for improving cartilage functions according to Aspect (1) or (2), wherein the lactoperoxidase, the cystatin, and the HMG-like protein are derived from bovine milk.
Aspect (4) The food product composition for improving cartilage functions according to any one of Aspects (1) to (3), wherein the degraded products of the lactoperoxidase, the cystatin, and the HMG-like protein are prepared through degradation of the lactoperoxidase, the cystatin, and the HMG-like protein in the presence of a protease.
Aspect (5) The food product composition for improving cartilage functions according to Aspect (4), wherein the protease is at least one protease selected from the group consisting of pepsin, trypsin, chymotrypsin, pancreatin, and papain.
Aspect (6) A drink or food product for improving cartilage functions comprising the food product composition for improving cartilage functions according to any one of Aspects (1) to (5), a nutritive composition for improving cartilage functions comprising the food product composition for improving cartilage functions according to any one of Aspects (1) to (5), or a livestock food for improving cartilage functions comprising the food product composition for improving cartilage functions according to any one of Aspects (1) to (5).
Aspect (7) A method of improving cartilage functions through daily ingestion of at least 1 mg of the lactoperoxidase, the cystatin, the HMG-like protein, and/or the degraded products thereof according to any one of Aspects (1) to (5).

### EFFECTS OF INVENTION

The food product composition for improving cartilage functions according to the present invention is useful in prevention and treatment of a variety of joint diseases because of its action of promoting proliferation of chondrocytes and/or retarding calcification of the chondrocytes and its noticeable effect of modifying or regenerating deformed or lost cartilages.

### BRIEF DESCRIPTION OF DRAWINGS

Fig. 1 is a graph illustrating the thicknesses of the articular cartilage layers of mouse right hindlimbs in six groups of mice fed for ten weeks with a standard food containing a standard level of phosphorus, a phosphorus-rich food, and inventive products, respectively.
Fig. 2 is a graph illustrating the number of chondrocytes present in the articular cartilage layers of mouse right hindlimbs in six groups of mice fed for ten weeks with a standard food containing a standard level of phosphorus, a phosphorus-rich food, and inventive products, respectively.

### DESCRIPTION OF EMBODIMENTS

The present inventors, who have conducted extensive research to solve the problems, have found that proteins contained in milk and dairy products, i.e., lactoperoxidase, cystatin, and high mobility group (HMG)-like protein and degraded products thereof promote the proliferation of chondrocytes and/or retard the calcification of the chondrocytes, and modify or regenerate deformed or lost cartilages, and have completed the present invention.

It has not been discovered that the proteins contained in milk and dairy products, i.e., lactoperoxidase, cystatin, HMG-like protein, and degraded products thereof promote the proliferation of chondrocytes and/or retard the calcification of the chondrocytes, and modify or regenerate deformed or lost cartilages.

The present invention is characterized in that the proteins contained in milk and dairy products, i.e., lactoperoxidase, cystatin, HMG-like protein and/or degraded products thereof are used as effective ingredients. The lactoperoxidase, cystatin, HMG-like protein used in the present invention can be those prepared from mammal milk, such as bovine milk, human milk, goat milk, or sheep milk, chemically synthetic products, those produced by gene engineering techniques, and those purified from blood or organs. Commercially available reagents of purified lactoperoxidase, cystatin, and HMG-like protein can also be used. The degraded products of lactoperoxidase, cystatin, and HMG-like protein used in the present invention can be prepared from lactoperoxidase, cystatin, and HMG-like protein treated with proteases.

Lactoperoxidase, one of the effective ingredients, is prepared by a known process of purifying milk using a polysaccharide-affinitive carrier having a sulfone group introduced thereinto (Japanese Patent Application Laid-Open No. 3-109400), for example. The degraded products of lactoperoxidase can be prepared from lactoperoxidase in the presence of proteases, such as trypsin, pancreatin, chymotrypsin, pepsin, papain, kallikrein, cathepsin, thermolysin, and V8 protease such that the molecular weight is 10,000 or less, preferably 500 or more.

Cystatin, another effective ingredient, is prepared by a known process of purifying milk, for example, by column chromatography (Japanese Patent Application Laid-Open No. 2000-281587). The degraded products of cystatin can be prepared from cystatin in the presence of proteases, such as trypsin, pancreatin, chymotrypsin, pepsin, papain, kallikrein, cathepsin, thermolysin, and V8 protease, such that the molecular weight is 8,000 or less, preferably 500 or more.

Another effective ingredient HMG-like protein is prepared by purifying milk by column chromatography (Japanese Patent Application Laid-Open No. 9-227403), for example. The degraded products of HMG-like protein can be prepared from HMG-like protein in the presence of proteases, such as trypsin, pancreatin, chymotrypsin, pepsin, papain, kallikrein, cathepsin, thermolysin, and V8 protease, such that the molecular weight is 6,000 or less, preferably 500 or more.

In the present invention, at least one effective ingredient selected from the group consisting of lactoperoxidase, cystatin, HMG-like protein, and degraded products thereof may be used as it is in the food product composition for improving cartilage functions according to the present invention. These proteins and their degraded products can be formulated into a drug in the form of powder, granules, tablets, capsules, or liquid according to a normal process when necessary.

In the present invention, the effective ingredients can be formulated into a drug or added to a drink or food product by any mixing or compounding process. For example, the effective ingredients can may be added and compounded in a solution as follows: At least one effective ingredient selected from the group consisting of lactoperoxidase, cystatin, HMG-like protein, and degraded products thereof are suspended or dissolved in deionized water, and are mixed with stirring. The solution is formulated into a drug or finished in the form of a drink or food product or a livestock food for use.

Mixing with stirring can be performed under any condition that can homogeneously mix effective ingredients. For example, the effective ingredients may be mixed with stirring with an ultrasonic disperser or a homomixer. The solution of the composition may be used after desalting or concentration through a reverse osmosis (RO) or ultrafiltration (UF) membrane or freeze drying, when necessary, such that the composition solution is readily formulated into a drug or used in a drink or food product or a livestock food. Sterilization processes usually used in production of pharmaceuticals, drink or food products, and livestock foods can be used in the present invention. The powder form can also be sterilized with dry heat.

The food product composition for improving cartilage functions according to the present invention can be formed into a variety of forms, such as liquid, gel, powder, and granules. The food product composition for improving cartilage functions according to the present invention after formulated into a drug can be compounded in drink or food products and nutritive compositions, such as nutritional supplements, yogurt, milk beverages, and wafers.

The food product composition for improving cartilage functions according to the present invention contains at least one effective ingredient selected from the group consisting of lactoperoxidase, cystatin, HMG-like protein, and degraded products thereof as effective ingredients, and can be formulated into a drug using a diluent or filler usually used, such as a filler, an extender, a binder, a disintegrant, a surfactant, and a lubricant. Examples of the filler include sucrose, lactose, starch, crystalline cellulose, mannite, light anhydrous silicic acid, aluminate magnesium, synthetic aluminum silicate, magnesium aluminometasilicate, calcium carbonate, sodium hydrogen carbonate, calcium hydrogen phosphate, and carboxylmethyl cellulose calcium. These fillers may be used alone or in combination.

The food product composition for improving cartilage functions according to the present invention may use a stabilizer, saccharides, lipids, flavorings, vitamins, minerals, flavonoids, and polyphenols in combination. These can be appropriately compounded in preparation of the drink or food product and the livestock food. These can also be used in combination with other ingredients improving the cartilage functions, such as glucosamine hydrochloride, and lactoferrin.

As shown in Experimental examples described below, in the food product composition for improving cartilage functions according to the present invention, at least one effective ingredient selected from the group consisting of lactoperoxidase, cystatin, HMG-like protein, and degraded products thereof may be orally administrated to mice at 1 mg/kg or more to promote the proliferation of chondrocytes and/or retard the calcification of chondrocytes. The ingestion dose for an experimental animal corresponds to an ingestion dose for an adult in terms of blood concentration of a drug ("Yakkohyouka (Evaluation of Drug Efficacy) vol. 8", Mitsuyoshi Nakashima (1993), Hirokawashoten, pp. 2-18). Usually, if an adult ingests at least one effective ingredient selected from the group consisting of lactoperoxidase, cystatin, HMG-like protein, and degraded products thereof at 1 mg or more daily, an improvement in cartilage functions, particularly, prevention and treatments of osteoarthritis can be expected.

Accordingly, at least one effective ingredient selected from the group consisting of lactoperoxidase, cystatin, and HMG-like protein, and degraded products thereof are formulated into a drug or compounded in a drink or food product such that its requisite amount can be ensured. For example, to achieve the ingestion of at least one effective ingredient selected from the group consisting of lactoperoxidase, cystatin, HMG-like protein, and degraded products thereof by an adult at 1 mg or more daily, depending on the form of the final product, such as a medicine, a drink or food product, or a livestock food, the final product may contain at least one effective ingredient selected from the group consisting of lactoperoxidase, cystatin, HMG-like protein, and degraded products thereof in a proportion of 0.0005% to 5% (weight/weight), preferably 0.05 to 2.5% (weight/weight) relative to the total mass.

The food product composition for improving cartilage functions can contain at least one effective ingredient selected from the group consisting of lactoperoxidase, cystatin, HMG-like protein, and degraded products thereof or in any combination these ingredients. Specific examples thereof include one of the ingredients; a combination of one of the three ingredients with its degraded product; a combination of two of the three ingredients, such as lactoperoxidase and cystatin, lactoperoxidase and HMG-like protein, and cystatin and HMG-like protein; a combination of one of the three ingredients with its degraded product; a combination of lactoperoxidase, cystatin, and HMG-like protein; and a combination of the three ingredients, at least one of which is its degraded product.

The food product composition for improving cartilage functions promotes the proliferation of chondrocytes and/or retards the calcification of chondrocytes. In other words, the food product composition for improving cartilage functions is useful for improvement in cartilage functions because of its noticeable effect of modifying or regenerating deformed or lost cartilages due to ageing, external injury, or load (overload) during exercise. Specifically, the food product composition for improving cartilage functions is useful for prevention and treatment of a variety of joint diseases, such as osteoarthritis and knee osteoarthritisl, in particular.

Examples of the drink or food products which can contain the food product composition for improving cartilage functions include, but should not be limited to, dairy products specified in Article 2 of "Ministerial Ordinance on Milk and Milk products Concerning. Compositional Standards, etc." of Japan (such as cheese, cream, butter, butter oil, ice cream, fermented milk, fermented milk drink, and milk drink), and food products based on these dairy products, and wafers. The fermented milk includes hard or solidified yogurt, soft or semi-fluid yogurt, and yogurt drink.

Examples and Experimental Examples of the present invention will now be described in detail. These are provided for illustrative purposes only, and should not be construed to limit the present invention.

### EXAMPLES

### [Example 1]

### (Preparation of lactoperoxidase)

A column (diameter: 5 cm, height: 30 cm) packed with a cationic exchange resin, sulfonated CHITO PEARL (made by Fujibo Holdings, Inc.) (400 g) was sufficiently washed with deionized water. Unpasteurized defatted milk (40 L, pH: 6.7) was fed through the column at a flow rate of 25 ml/min. After the feed, the column was sufficiently washed with deionized water, and elution was performed with 0.02 M carbonic acid buffer solution (pH: 7.0) containing 2.0 M sodium chloride. The eluted fraction containing lactoperoxidase was adsorbed onto a S-Sepharose FF column (made by GE Healthcare, Inc.), was sufficiently washed with deionized water, and was equilibrated with 10 mM phosphoric acid buffer solution (pH: 7.0). The adsorbed fraction was eluted by a linear gradient of 0 to 2.0 M sodium chloride to recover a fraction containing lactoperoxidase. The fraction was then treated by gel filtration chromatography using HiLoad 16/60 Superdex 75 pg (made by GE Healthcare, Inc.) to give lactoperoxidase (3.0 g). The lactoperoxidase had a purity of 94%. The lactoperoxidase can be used as it is in a food product composition for improving cartilage functions (cartilage function improver/Inventive product 1).

### [Example 2]

### (Preparation of degraded lactoperoxidase products)

The lactoperoxidase (1 g) prepared in Example 1 was dissolved in water (200 ml), and a protease pancreatin (made by Sigma-Aldrich Corporation) was added such that the final concentration was 0.01% by weight. The solution was subjected to an enzyme treatment at 37°C for five hours. The enzyme was deactivated by a heat treatment at 90°C for five minutes. The solution was freeze-dried to give a degraded lactoperoxidase product (0.8 g). The degraded lactoperoxidase product had a molecular weight of 10,000 or less. This product can be used as it is in a food product composition for improving cartilage functions (cartilage function improver/Inventive product 2).

### [Example 3]

### (Preparation of degraded lactoperoxidase product)

The lactoperoxidase (1 g) prepared in Example 1 was dissolved in water (200 ml), and a protease trypsin (made by Sigma-Aldrich Corporation) was added such that the final concentration was 0.01% by weight. The solution was subjected to an enzyme treatment at 37 °C for five hours. The enzyme was deactivated by a heat treatment at 90°C for five minutes, and was freeze-dried to give a degraded lactoperoxidase product (0.9 g). The degraded lactoperoxidase product had a molecular weight of 10,000 or less. This product can be used as it is in a food product composition for improving cartilage functions (cartilage function improver/Inventive product 3).

### [Example 4]

### (Preparation of cystatin)

A column packed with S Sepharose (3,000 g) was sufficiently washed with deionized water. Defatted milk (10,000 L) was fed through the column. The column was sufficiently washed with deionized water, and elution was performed by a linear gradient of 0.1 to 1.0 M sodium chloride. The eluted fraction was subjected to a heat treatment at 90°C for ten minutes, and sediments were centrifugally removed. The eluted fraction containing cystatin was again fractionated by Mono S ion exchange chromatography. The fraction was sequentially treated in a FPLC system by Mono Q ion exchange chromatography, and then by Superose 12 gel filtration chromatography, and in an HPLC system by hydroxyapatite chromatography, and C4 reversed-phase chromatography to give cystatin (58 mg). The cystatin can be used as it is in a food product composition for improving cartilage functions (cartilage function improver/Inventive product 4).

### [Example 5]

### (Preparation of cystatin)

A 5% whey protein solution (10,000 L) was subjected to a heat treatment at 90°C for ten minutes, and sediments were centrifugally removed. A carrier of carboxymethylated papain bound to Tresyl-Toyopearl, made by Tosoh Corporation) was packed into a column. The system was equilibrated with a 0.5 M sodium chloride solution. The whey protein solution was fed through the column. After the feed, the system was washed with a 0.5 M sodium chloride solution and then a 0.5 M sodium chloride solution containing 0.1% Tween20. In the next step, a cysteine protease was eluted with a 20 mM acetic acid-0.5 M sodium chloride solution. The eluted fraction was immediately neutralized with a 1 M sodium hydroxide solution, and was fractionated through sequential treatment by Mono S cationic exchange chromatography and by hydroxyapatite chromatography and C4 reversed-phase chromatography in an HPLC system to give cystatin (48 mg). The cystatin can be used as it is in a food product composition for improving cartilage functions (cartilage function improver/Inventive product 5).

### [Example 6]

### (Preparation of degraded cystatin product)

The cystatin (25 mg) prepared in Example 4 was dissolved in water (100 ml), and pancreatin (made by Sigma-Aldrich Corporation) was added such that the final concentration was 1%. The solution was subjected to an enzyme treatment at 37°C for five hours. The enzyme was deactivated by an heat treatment at 90°C for five minutes, and was freeze-dried to give a degraded cystatin product (Inventive product 6A) (23 mg). The cystatin (25 mg) prepared in Example 5 was treated as described above to give a degraded cystatin product (Inventive product 6B) (24 mg). The degraded cystatin products had a molecular weight of 8,000 or less. The products can be used as they are in food product compositions for improving cartilage functions (cartilage function improvers/Inventive product 6A and Inventive product 6B).

### [Example 7]

### (Preparation of HMG-like protein)

Acolumn packed with sulfonated CHITO PEARL (3,000 g) (made by Fujibo Holdings, Inc.) was sufficiently washed with deionized water. Defatted milk (300 L) was fed through the column. The column was then sufficiently washed with deionized water. Basic protein was adsorbed onto the resin with a 0.02 M carbonic acid buffer solution (pH: 6.7), and was eluted by a linear gradient of 0.1 M to 1.0 M NaCl to be recovered. In the next step, the eluted fraction containing HMG-like protein was eluted by a linear gradient of 0.1 M to 1.0 M NaCl in S-Sepharose cationic exchange chromatography where the system was equilibrated with a 0.1 M phosphoric acid buffer solution (pH: 6.5). The eluted fraction was subjected to a heat treatment at 90°C for ten minutes, and sediments were centrifugally removed. The fraction was further fractionated by a linear gradient of 0.1 M to 1.0 M NaCl in Mono Q ion exchange chromatography where the system was equilibrated with a 0.1 M phosphoric acid buffer solution (pH: 6.5), and then was fractionated by Sepharose 12 gel filtration chromatography where the system was equilibrated with a 0.1 M phosphoric acid buffer solution (pH: 6.5). The fraction was finally treated with a high performance liquid chromatograph using a C4 reversed-phase column to give HMG-like protein (135 mg). The HMG-like protein can be used as it is in a food product composition for improving cartilage functions (cartilage function improver/Inventive product 7).

### [Example 8]

### (Preparation of degraded HMG-like protein product)

The HMG-like protein (25 mg) prepared in Example 7 was dissolved in water (100 ml), and a protease trypsin (made by Sigma-Aldrich Corporation) was added such that the final concentration was 0.01% by weight. The solution was subjected to an enzyme treatment at 37°C for five hours. The enzyme was deactivated through a heat treatment at 90°C for five minutes. The solution was freeze-dried to give a degraded HMG-like protein product (24 mg). The degraded HMG-like protein product had a molecular weight of 6,000 or less. The product can be used as it is in a food product composition for improving cartilage functions (cartilage function improver/Inventive product 8).

### [Experimental Example 1]

### (Cell experiment)

The action of promoting the proliferation of chondrocytes was evaluated about lactoperoxidase of Inventive product 1, the degraded lactoperoxidase product of Inventive product 2, cystatin of Inventive product 4, the degraded cystatin product of Inventive product 6B, HMG-like protein of Inventive product 7, the degraded HMG-like protein product of Inventive product 8, a mixture of Inventive products 1 and 4, and a mixture of Inventive products 5 and 7. Mouse-derived cartilage progenitor cell strains (ATDC5) were seeded at a density of 5×10³ cells/well in a 96-well cell culturing plate, and were incubated in Dulbecco's Modified Eagle medium/Ham's nutrient mixture F-12 containing 5% fetal bovine serum with an incubator for 24 hours at 37°C under a 5% CO₂ atmosphere. The medium was then completely replaced with new one. Inventive products 1, 2, 4, 6B, 7, and 8, the mixture of Inventive products 1 and 4, and the mixture of Inventive products 5 and 7 dissolved in PBS(-) were each added to the medium such that the final concentration was 1 µg/mL, followed by incubation for 48 hours. PBS(-), which was used as a solvent, was used as a negative control. The medium was completely removed. A cell proliferation reagent WST-1 (made by Roche Diagnostics GmbH) was added to the medium at a content of 1/10 of the medium, and the samples were incubated in the resulting medium for six hours. The absorbance of each sample was measured with a plate reader at 440 nm. Since WST-1 is reduced into a formazan dye by the metabolic activity of living cells, the amount of the formazan dye is proportional to the number of cells having metabolic activity. The absorbance reflecting the amount of the formazan dye was used as an index indicating the promotion of the proliferation of chondrocytes. The results are shown in Table 1.

**[Table 1]**

| | Absorbance (440 nm) |
|---|---|
| PBS(-) | 0.616 ± 0.021 |
| Inventive product 1 | 0.753 ± 0.015* |
| Inventive product 2 | 0.723 ± 0.009* |
| Inventive product 4 | 0.885 ± 0.021* |
| Inventive product 6B | 0.743 ± 0.017* |
| Inventive product 7 | 0.819 ± 0.017* |
| Inventive product 8 | 0.771 ± 0.008* |
| Inventive products 1 +4 | 0.853 ± 0.023* |
| Inventive products 5+7 | 0.760 ± 0.014* |

| | |
|---|---|
| The value indicates an average ± a standard deviation (n=5). * represents a significant difference from PBS(-) (p<0.05). | |

These results demonstrate that the number of cartilage progenitor cells significantly increased in the media containing lactoperoxidase of Inventive product 1, the degraded lactoperoxidase product of Inventive product 2, cystatin of Inventive product 4, the degraded cystatin product of Inventive product 6B, HMG-like protein of Inventive product 7, the degraded HMG-like protein product of Inventive product 8, the mixture of Inventive products 1 and 4, and the mixture of Inventive products 5 and 7, compared to the medium containing PBS(-). Consequently, the lactoperoxidase, cystatin, HMG-like protein, and degraded products thereof according to the present invention promoted the proliferation of chondrocytes.

### [Experimental Example 2]

### (Cell experiment)

The action of retarding the calcification of chondrocytes was evaluated about lactoperoxidase of Inventive product 1, the degraded lactoperoxidase product of Inventive product 3, cystatin of Inventive product 5, the degraded cystatin product of Inventive product 6A, HMG-like protein of Inventive product 7, the degraded HMG-like protein product of Inventive product 8, a mixture of Inventive products 1 and 7, and a mixture of Inventive products 4 and 8. Mouse-derived cartilage progenitor cell strains (ATDC5) were seeded at a density of 3×10³ cells/well in a 96-well cell culturing plate, and were incubated in a Dulbecco's Modified Eagle medium/Ham's nutrient mixture F-12 containing 5% fetal bovine serum with an incubator for 24 hours at 37°C under a 5% CO₂ atmosphere. The medium was then completely replaced with new one. Inventive products 1, 3, 5, 6A, 7, and 8, the mixture of Inventive products 1 and 7, and the mixture of Inventive products 4 and 8 dissolved in PBS(-) were each added to the medium such that the final concentration was 1 µg/mL. Cells were incubated for seven days while the medium was replaced every day. PBS(-), which was used as a solvent, was used as a negative control. After completion of the incubation, cells were fixed with a 20% formalin solution for 20 minutes, and were washed with water. Water was added, and the cells were left to stand for 15 minutes. Naphthol AS-BI phosphate and Fast Red Violet LB Salt were dissolved in a 0.05 M 2-amino-2-methyl-1,3-propanediol (AMP) solution to prepare an alkali phosphatase (ALP) dyeing solution, and cells were dyed with the ALP dyeing solution for 20 minutes at 37°C. After the dyeing, cells were washed with water five times, and were air dried. From an image of dyed cells in the culturing plate taken in with a scanner, the area of the dyed cells was calculated with image processing software. The area of the dyed cells is represented as a relative phase to the value of 1 in the negative control. The ALP activity is an initial marker of calcification in chondrocytes, and a smaller area of the dyed cells indicates that the calcification is more retarded. The results are shown in Table 2.

**[Table 2]**

| | Relative value of area of dyed cells |
|---|---|
| PBS(-) | 1.00 ± 0.08 |
| Inventive product 1 | 0.20 ± 0.08* |
| Inventive product 3 | 0.34 ± 0.05* |
| Inventive product 5 | 0.58 ± 0.04* |
| Inventive product 6A | 0.61 ± 0.07* |
| Inventive product 7 | 0.63 ± 0.06* |
| Inventive product 8 | 0.70 ± 0.08* |
| Inventive products 1 +7 | 0.50 ± 0.06* |
| Inventive products 4+8 | 0.47 ± 0.05* |

| | |
|---|---|
| The value represents an average ± a standard deviation (n=5). * represents a significant difference from PBS(-) (p<0.05). | |

The results demonstrate that the ALP-dyed area significantly increases in the media containing lactoperoxidase of Inventive product 1, degraded lactoperoxidase product of Inventive product 3, cystatin of Inventive product 5, degraded cystatin product of Inventive product 6A, HMG-like protein of Inventive product 7, degraded HMG-like protein product of Inventive product 8, the mixture of Inventive products 1 and 7, and the mixture of Inventive products 4 and 8. Consequently, the lactoperoxidase, cystatin, and HMG-like protein, and degraded products thereof according to the present invention retarded the calcification of chondrocytes, compared to the medium containing PBS(-).

### [Experimental Example 3]

### (Animal experiment)

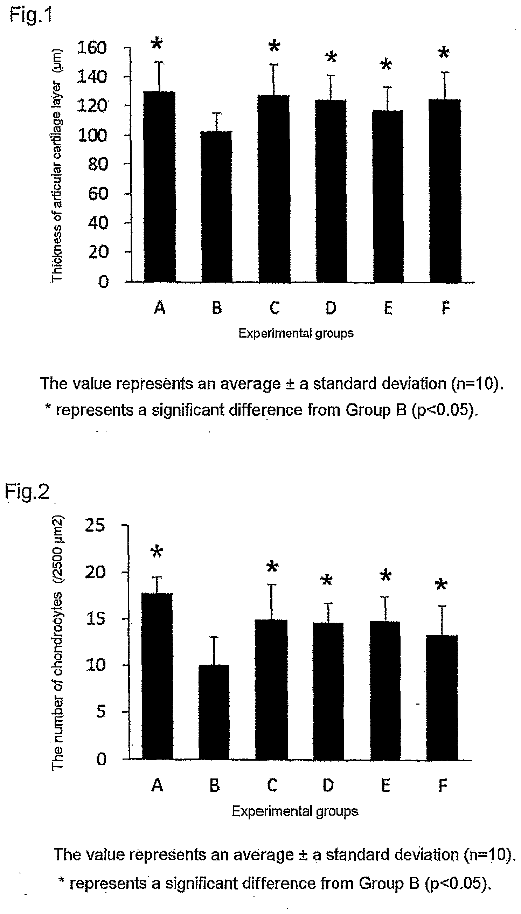

The effects of lactoperoxidase of Inventive product 1, the degraded lactoperoxidase product of Inventive product 3, cystatin of Inventive product 4, and HMG-like protein of Inventive product 7 were examined about the initial degeneration of the articular cartilage layer. Ten-week C57BL/6J male mice were divided into six groups of ten mice such that their weights were not varied among the groups. The group of mice ingesting a livestock food (standard food) containing a standard level of phosphorus (0.2 g/100 g livestock food) without Inventive products was defined as Group A, and the group ingesting a livestock food (phosphorus-rich food) containing a large amount of phosphorus (1.2 g/100 g livestock food) without Inventive products was defined as Group B. The group ingesting Inventive product 1 was defined as Group C, the group ingesting Inventive product 4 as Group D, the group ingesting Inventive product 7 as Group E, and the group ingesting Inventive product 3 as Group F. Each Inventive product was compounded with the phosphorus-rich food such that the ingestion dose of Inventive product per day was 1 mg/kg of a mouse. After feeding for ten weeks, right hindlimbs were extracted, and were immersed in a 10% neutral buffer formalin solution to be fixed. Paraffin-embedded sections of knee joints were prepared, and were subjected to haematoxylin-eosin dyeing. Using the dyed samples, the thickness of the articular cartilage layer and the number of chondrocytes present in the articular cartilage layer were measured to evaluate the effect on the initial degeneration in the articular cartilage layer. The results are shown in Figs. 1 and 2.

The results demonstrate that in Group B ingesting the phosphorus-rich food without Inventive product, the thickness of the articular cartilage layer and the number of chondrocytes present in the articular cartilage layer significantly reduced due to excess ingestion of phosphorus, compared to Group A ingesting the standard food without Inventive product. In contrast, in Groups C to F ingesting Inventive products 1, 3, 4, and 7, decreases in the thickness of the articular cartilage layer and the number of chondrocytes were more significantly retarded than in Group B. Consequently, the lactoperoxidase, cystatin, and HMG-like protein, and degraded products thereof according to the present invention retarded the initial degeneration of the articular cartilage.

### [Example 9]

### (Preparation of tablets for improving cartilage functions)

Raw materials were mixed in the proportion shown in Table 3. The mixture was molded into 1 g of tablet for improving cartilage functions by a normal process. The tablet (1 g) contained 1 mg of lactoperoxidase of Inventive product 1.

**[Table 3]**

| | |
|---|---|
| Hydrous crystal glucose | 93.4 (% by weight) |
| Inventive product 1 | 0.1 |
| Mixed minerals | 5.0 |
| Sugar ester | 1.0 |
| Fragrance | 0.5 |

### [Example 10]

### (Preparation of nutritive composition for improving cartilage functions)

A degraded lactoperoxidase product of Inventive product 3 (0.5 g) was dissolved in deionized water (4,999.5 g), and was heated to 45°C. The materials were mixed under stirring with a TK homomixer (TK ROBO MICS; made by Tokushu Kika Kogyo Co., Ltd.) at 6,000 rpm for 30 minutes to prepare a solution containing 0.5 g/5.0 kg of degraded lactoperoxidase product of Inventive product 3. Raw materials shown in Table 4 were added to the solution (5.0 kg), and were further mixed with stirring for ten minutes. The mixture was placed into a 200 ml retort pouch, and was sterilized with a retort sterilizer (class 1 pressure vessel, type: RCS-4 CRTGN, made by HISAKA WORKS, LTD.) at 121 °C for 20 minutes to prepare 50 kg of a liquid nutritive composition for improving cartilage functions according to the present invention. The liquid nutritive composition for improving cartilage functions contained 1 mg of degraded lactoperoxidase product of Inventive product 3 relative to 100 g of the liquid nutritive composition.

**[Table 4]**

| | |
|---|---|
| Casein | 5.0 (kg) |
| Soybean protein | 5.0 |
| Fish oil | 1.0 |
| Shiso oil | 3.0 |
| Dextrin | 17.0 |
| Mixed minerals | 6.0 |
| Mixed vitamins | 1.95 |
| Emulsifier | 2.0 |
| Stabilizer | 4.0 |
| Fragrance | 0.05 |

### [Example 11]

### (Preparation of beverage for improving cartilage functions)

Skim milk powder (140 g) was dissolved in deionized water (569.95 g). Cystatin of Inventive product 4 (0.05 g) was dissolved. The system was heated to 45°C, and was mixed under stirring with an ultrasonic disperser (ULTRA-TURRAX T-25; made by IKA Japan K.K) at 9,500 rpm for 30 minutes. Raw materials shown in Table 5 were added, and were mixed with stirring for 15 minutes. The mixture was placed into 100 ml glass bottles, and was sterilized at 95°C for 15 seconds. The bottles were sealed to prepare ten bottles of beverage (100 ml) for improving cartilage functions according to the present invention. The beverage for improving cartilage functions contained 5 mg of cystatin of Inventive product 4 relative to 100 ml of the beverage.

**[Table 5]**

| | |
|---|---|
| Maltitol | 100 (g) |
| Acidulant | 2 |
| Reduced starch syrup | 20 |
| Fragrance | 2 |
| Deionized water | 166 |

### [Example 12]

### (Preparation of livestock food for improving cartilage functions for dog)

HMG-like protein of Inventive product 7 (0.1 g) was dissolved in deionized water (99.9 g). The system was heated to 45°C, and was mixed with an ultrasonic disperser (ULTRA-TURRAX T-25; made by IKA Japan. K.K.) at 9,500 rpm for 40 minutes to prepare a solution containing 0.1 g/100 g of HMG-like protein of Inventive product 7. Raw materials shown in Table 6 were added to the solution (100 g), and were further mixed with stirring for 40 minutes. The mixture was sterilized at 120°C for 4 minutes to prepare 1 kg of a livestock food for improving cartilage functions for dogs according to the present invention. The livestock food for improving cartilage functions for dogs contained 10 mg of HMG-like protein of Inventive product 7 relative to 100 g of the livestook food.

**[Table 6]**

| | |
|---|---|
| Soybean meal | 120 (g) |
| Skim milk powder | 140 |
| Soybean oil | 40 |
| Corn oil | 20 |
| Palm oil | 232 |
| Corn starch | 140 |
| Wheat flour | 90 |
| Bran | 20 |
| Mixed vitamins | 50 |
| Cellulose | 28 |
| Mixed minerals | 20 |

The invention is further described with reference to the following numbered paragraphs:
1. A food product composition for improving cartilage functions, comprising at least one effective ingredient selected from the group consisting of lactoperoxidase, cystatin, HMG-like protein, and degraded products thereof.
2. As paragraph 1, wherein the lactoperoxidase, the cystatin, and the HMG-like protein are derived from mammal milk.
3. As paragraph 1 or 2, wherein the lactoperoxidase, the cystatin, and the HMG-like protein are derived from bovine milk.
4. As any of paragraphs 1 to 3, wherein the degraded products of the lactoperoxidase, the cystatin, and the HMG-like protein are prepared through degradation of the lactoperoxidase, the cystatin, and the HMG-like protein in the presence of a protease.
5. As paragraph 4, wherein the protease is at least one protease selected from the group consisting of pepsin, trypsin, chymotrypsin, pancreatin, and papain.
6. A drink or food product for improving cartilage functions comprising the food product composition for improving cartilage functions according to any one of paragraphs 1 to 5, a nutritive composition for improving cartilage functions comprising the food product composition for improving cartilage functions according to any one of paragraphs 1 to 5, or a livestock food for improving cartilage functions comprising the food product composition for improving cartilage functions according to any one of paragraphs 1 to 5.
7. A method of improving cartilage functions through daily ingestion of at least 1 mg of the lactoperoxidase, the cystatin, the HMG-like protein, and/or the degraded products thereof according to any one of paragraphs 1 to 5.

## Claims

1. A food product composition for improving cartilage functions, comprising at least one effective ingredient selected from the group consisting of cystatin, HMG-like protein, and degraded products thereof.

2. The food product composition for improving cartilage functions according to claim 1, wherein the cystatin, and the HMG-like protein are derived from mammal milk.

3. The food product composition for improving cartilage functions according to claim 1 or 2, wherein the cystatin, and the HMG-like protein are derived from bovine milk.

4. The food product composition for improving cartilage functions according to any one of claims 1 to 3, wherein the degraded products of the cystatin, and the HMG-like protein are prepared through degradation of the cystatin, and the HMG-like protein in the presence of a protease.

5. The food product composition for improving cartilage functions according to claim 4, wherein the protease is at least one protease selected from the group consisting of pepsin, trypsin, chymotrypsin, pancreatin, and papain.

6. A drink or food product for improving cartilage functions comprising the food product composition for improving cartilage functions according to any one of claims 1 to 5, a nutritive composition for improving cartilage functions comprising the food product composition for improving cartilage functions according to any one of claims 1 to 5, or a livestock food for improving cartilage functions comprising the food product composition for improving cartilage functions according to any one of claims 1 to 5.
